# EUROPEAN PATENT SPECIFICATION

(11) **EP 3 332 700 B1**
(45) Date of publication and mention of the grant of the patent: **26.02.2020**
(21) Application number: 16844123.6
(22) Date of filing: 15.08.2016
(51) Int. Cl.: A61B 5/02

(54) **PULSE WAVE DETECTOR**
PULSWELLENDETEKTOR
DÉTECTEUR D'ONDE DE POULS

(30) Priority: 07.09.2015 JP 2015175966
(43) Date of publication of application: 13.06.2018
(73) Proprietor: Omron Healthcare Co., Ltd., Muko-shi, Kyoto 617-0002 (JP)
(72) Inventor: BRIGHAM Brian, Muko-shi Kyoto 617-0002 (JP); FUKUTSUKA Masayuki, Muko-shi Kyoto 617-0002 (JP); YAMASHITA Shingo, Muko-shi Kyoto 617-0002 (JP); KITAGAWA Tsuyoshi, Muko-shi Kyoto 617-0002 (JP); OGURA Toshihiko, Muko-shi Kyoto 617-0002 (JP); WAKAMIYA Masayuki, Muko-shi Kyoto 617-0002 (JP)
(74) Representative: Isarpatent
(86) International application number: PCT/JP2016/073851
(87) International publication number: WO 2017/043260

(56) References cited:
- JP-A- H03 146 027
- JP-A- 2004 222 931
- JP-A- 2005 087 483
- JP-A- 2008 168 054
- US-A- 5 467 771
- US-A1- 2003 149 369

## Description

### Technical Field

The present invention relates to a pulse wave detecting device.

### Background Art

A biological information measuring device is known that, in a state where a pressure sensor is directly contacted with a living body portion through which an artery such as the radial artery in the wrist passes, can measure biological information such as the pulse and the blood pressure by using information detected by the sensor (for example, see Patent Literatures 1 to 3).

Patent Literature 1 discloses a biological information measuring device in which an opening for avoiding the ulna is disposed in a portion that is to be wound around the back side of the hand in a state where the device is attached to the wrist, whereby the attachability of the device to the wrist is improved.

Patent Literature 2 discloses a biological information measuring device in which a band that is to be wound around the wrist, and that is stretchable and contractible in the winding direction is provided with: a marking portion that stretches or contracts in connection with stretching or contraction of the band, and that indicates the band wound strength depending on the degree of stretching or contraction of the band; and a reference button functioning as a reference for determining whether the degree of stretching or contraction indicated by the marking portion corresponds to the optimum band wound strength or not.

According to the biological information measuring device, when the attachment of the device is performed by using the marking portion and the reference button, the device can be attached at the optimum band wound strength irrespective of the diameter of the wrist of the user.

Patent Literature 3 discloses a biological information measuring device in which both end portions of a band portion that is to be wound around the wrist are divided into three parts, and one and other ends of the divided band parts are enabled to be respectively connected to each other.

### Citation List

### Patent Literature

Patent Literature 1: JP-A-2008-168054
Patent Literature 2: JP-A-05-329117
Patent Literature 3: JP-A-51-041285

US 5,467,771 A describes a pulse wave sensor for detecting a pressure pulse wave produced from an arterial vessel of a living subject.

US 2003/149369 A1 describes a method and an apparatus for measuring hemodynamic parameters using parametrics.

JP H03 146027 A describes a fitting device for a pulse wave detecting probe.

### Summary of Invention

### Technical Problem

Each of the biological information measuring devices disclosed in Patent Literatures 1 to 3 is to be attached to the wrist by, in a state where the pressure sensor portion is butted against the vicinity of the radial artery to be held, winding the band toward the back side of the hand, and then fixing it.

The styloid process of the radius exists in the vicinity of the radial artery. In the state where the pressure sensor portion is butted against the vicinity of the radial artery to be held, a hard portion of the styloid process of the radius hits against the housing of the device.

When the housing of the device has a high rigidity, as a result, the housing easily enters an unstable state. When, in this state, the winding or the like of the band is performed, and the device is fixed to the wrist, there is a possibility that the pressure sensor portion is fixed at a position which is displaced from a desired position. Moreover, the winding of the band is performed in an unstable state, and therefore the attachment itself of the device is not easily performed.

Patent Literature 1 discloses an example in which a housing portion which is supposed to butt against the styloid process of the radius is configured by a high rigidity material. In this configuration, however, the housing is caused to easily enter an unstable state, by an influence of the radius. Patent Literature 1 discloses another example in which the whole housing is configured by a low rigidity material. In this configuration, the housing is hardly caused to enter an unstable state by the styloid process of the radius. In the case where the whole housing which accommodates the pressure sensor has a low rigidity, after the device is attached, however, the position of the pressure sensor is easily displaced by an external impact or a motion of the user.

In each of the biological information measuring devices disclosed in Patent Literatures 2 and 3, the pressure sensor is fixed to the band having a low rigidity. After the device is attached, therefore, the position of the pressure sensor is easily displaced by an external impact or a motion of the user.

The invention has been conducted in view of the above circumstances. It is an object of the invention to provide a pulse wave detecting device in which the attachability can be improved while preventing the position of a pulse wave detection sensor from being displaced.

### Solution to Problem

A pulse wave detecting device of the invention is defined in claim 1.

### Advantageous Effects of Invention

According to the invention, it is possible to provide a pulse wave detecting device in which the attachability can be improved while preventing the position of a pulse wave detection sensor from being displaced.

### Brief Description of Drawings

[Fig. 1] Fig. 1 is a diagram showing the external configuration of a housing 20 of a biological information measuring device of the wrist wearing type for illustrating an embodiment of the invention.
[Fig. 2] Fig. 2 is a diagram of the housing 20 shown in Fig. 1, as seen from the side which is to be in contact with the wrist.
[Fig. 3] Fig. 3 is a cross-sectional diagram taken along line A-A in Fig. 2.
[Fig. 4] Fig. 4 is a view illustrating a method of attaching the biological information measuring device to the wrist.
[Fig. 5] Fig. 5 is a cross-sectional diagram of a housing 20A which is a modification of the housing 20.
[Fig. 6] Fig. 6 is a cross-sectional diagram of a housing 20B which is a modification of the housing 20.
[Fig. 7] Fig. 7 is a cross-sectional diagram of a housing 20C which is a modification of the housing 20.

### Description of Embodiments

Hereinafter, an embodiment of the invention will be described with reference to the drawings.

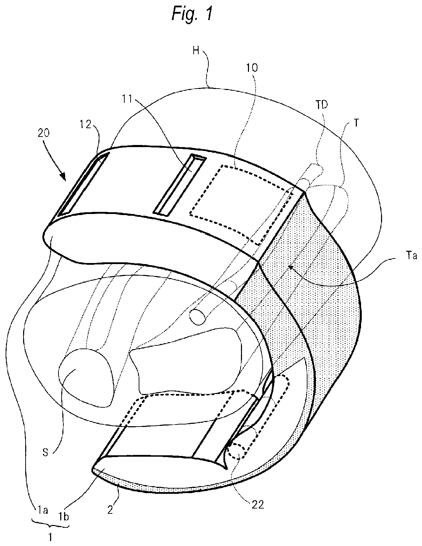

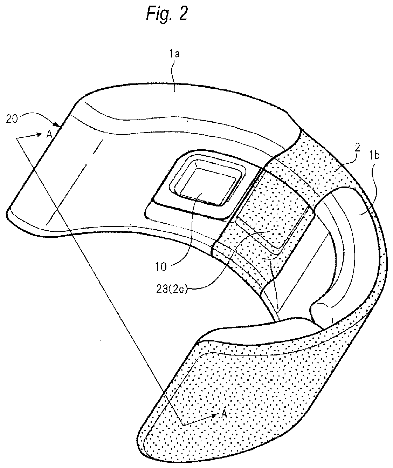

Fig. 1 is a diagram showing the external configuration of a housing 20 of a biological information measuring device of the wrist wearing type for illustrating an embodiment of the invention. Fig. 2 is a diagram of the housing 20 shown in Fig. 1, as seen from the side which is to be in contact with the wrist.

Fig. 1 shows the left wrist H of the subject. The near side of the figure coincides with the direction in which the hand of the subject exists. The upper side of the figure is in the direction along which the palm of the hand is oriented. In the wrist H, the radius T, the ulna S, and the radial artery TD are shown.

The biological information measuring device of the embodiment has a pulse wave detector 10 which detects a pulse wave (a pressure pulse wave or a volume pulse wave) from the radial artery TD that extends along the radius T of the wrist H of the subject, and measures biological information such as the blood pressure value and the pulse rate based on pulse waves detected by the pulse wave detector 10.

The pulse wave detector 10 may have a known configuration. For example, the pulse wave detector 10 has a pressure sensor functioning as the pulse wave detection sensor, and a mechanism which presses it against the skin, and detects a pressure pulse wave by using the pressure sensor. Alternatively, the pulse wave detector 10 has a photoelectric sensor functioning as the pulse wave detection sensor, and detects a volume pulse wave from a signal detected by the photoelectric sensor.

The biological information measuring device includes: a housing 20 which accommodates: the pulse wave detector 10; and a biological information calculator that is not shown, and that calculates biological information such as the blood pressure value and the pulse rate based on the pulse wave detected by the pulse wave detector 10; and a band which is used for fixing the housing 20 to the wrist, and which will be described later.

The biological information measuring device is requested to have at least the pulse wave detector 10, and functions as the pulse wave detecting device. For example, the biological information calculator may be disposed in an apparatus other than the biological information measuring device.

The housing 20 is configured so as to be woundable in the circumferential direction of the wrist H, and to be attached to the wrist H while covering the styloid process Ta of the radius T. The housing 20 has an approximately U-like shape which is opened on the side of the ulna S of the wrist H, and is configured so that the portion between the both ends in the circumferential direction of the wrist H does not cover the ulna S.

The housing 20 includes: a rigid portion 1 (the first housing portion) including a rigid portion 1a (the first sub-housing portion) and a rigid portion 1b (the second sub-housing portion); and a flexible portion 2 (the second housing portion) which is lower in rigidity than the rigid portion 1.

Each of the rigid portion 1a and the rigid portion 1b, and the flexible portion 2 are coupled together by fixation such as adhesion or welding, or by coupling pins.

The rigid portion 1a accommodates the pulse wave detector 10. The rigid portion 1a is configured by a first material having a high rigidity in order to stabilize the position of the pulse wave detector 10 with respect to the radial artery TD in the state where the housing 20 is attached to the wrist H, and to protect the pulse wave detector 10 including precision elements. As the first material, for example, a resin or a metal is used.

The flexible portion 2 is configured by a second material which is lower in rigidity than the first material. As the second material, for example, an elastic member, a shape-memory alloy, or the like is used. Particularly preferably, an elastomer, rubber, or a sponge is used as the second material.

The rigid portion 1b is configured by the first material in the same manner as the rigid portion 1a, but alternatively may be configured by a material which is different from the first material (however, a material which is higher in rigidity than the second material).

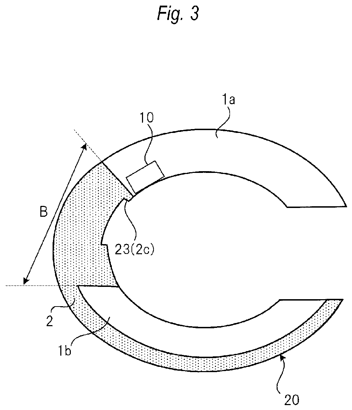

Fig. 3 is a cross-sectional diagram taken along line A-A in Fig. 2. In Fig. 3, some components are not illustrated.

As shown in Fig. 3, the rigid portion 1a and the flexible portion 2 are coupled to each other while being juxtaposed in the circumferential direction of the wrist H.

In a range B in the vicinity of a portion coupled to the rigid portion 1a, the flexible portion 2 has a thickness which is approximately equal to that of the rigid portion 1a.

A portion of the flexible portion 2 which is on the tip end side with respect to the range B is thinner than the range B. The portion of the flexible portion 2 which has the reduced thickness constitutes the outer circumferential surface (the surface opposite to the inner circumferential surface which is to be in contact with the wrist H) of the housing 20.

On the surface which is in the portion of the range B in the flexible portion 2, and which is on the side of the wrist H, there is a region 2c with which the styloid process Ta of the radius T of the subject is to be contacted in the case where the housing 20 is wound around the wrist H by a method specified by the manufacturer. In Figs. 1, 2, and 3, a recess 23 is formed in the surface which is in the portion of the range B in the flexible portion 2, and which is on the side of the wrist H, and the bottom surface of the recess 23 is used as it is as the region 2c.

In the region 2c with which the styloid process Ta is contacted in the state where the housing 20 is attached to the wrist, namely, the recess 23 having the plan view area which is equal to that of the region 2c in a plan view is formed. The range B is requested to be a range including at least the region 2c in the circumferential direction of the wrist.

The region of the housing 20 with which the styloid process Ta is contacted in the state where the housing 20 is attached to the wrist is varied depending on the shape of the wrist of the subject. When, with respect to every supposable wrist shape, the region of the housing 20 with which the styloid process Ta is to be contacted is simulated, therefore, it is possible to determine the region 2c with which the styloid processes Ta of many unspecified subjects may be possibly contacted.

In the biological information measuring device of the embodiment, the housing 20 is designed so that at least the region 2c is configured by the flexible portion 2.

In the thickness direction of the housing 20, the rigid portion 1b overlaps with the portion of the flexible portion 2 excluding the range B, and constitutes a part of the inner circumferential surface of the housing 20.

As shown in Fig. 1, a band fastener 22 for fixing a band (not shown) which is used for fixing the housing 20 to the wrist H is disposed on the inner circumferential surface of the housing 20. In the example of Fig. 1, the band fastener 22 is configured by a columnar metal fitting. The basal end of the band is fixed to the band fastener 22.

The housing 20 shown in Fig. 1, and the band constitute the biological information measuring device. In a configuration where the housing 20 can be fixed to the wrist without using a band, however, the housing 20 itself constitutes the biological information measuring device.

In the outer circumferential surface (the surface opposite to the surface opposed to the wrist H) of the rigid portion 1a, hole portions 11, 12 for engaging the tip end of the band with the rigid portion 1a are disposed in juxtaposition with each other in the circumferential direction of the wrist H.

The band is configured by a member having a belt-like shape which extends in the longitudinal direction (synonymous with the circumferential direction of the wrist H) of the housing 20, and which is lower in rigidity than the housing 20. As the member, for example, cloth or leather is used. A hook and loop fastener for affixing a portion of the band to another portion is disposed on the band.

In the biological information measuring device of the embodiment, in the state of Fig. 1, the subject causes the tip end portion of the band which is fixed to the band fastener 22, to pass through the hand palm side, inserts the band into the hole portion 12, and then pulls out the band from the hole portion 11.

Thereafter, the subject pulls the band which is taken out from the hole portion 11 toward the back of the hand, to adjust the fastening state, and then causes the band portions to be engaged with each other by the hook and loop fastener, thereby completing the fixing of the housing 20 to the wrist H by the band.

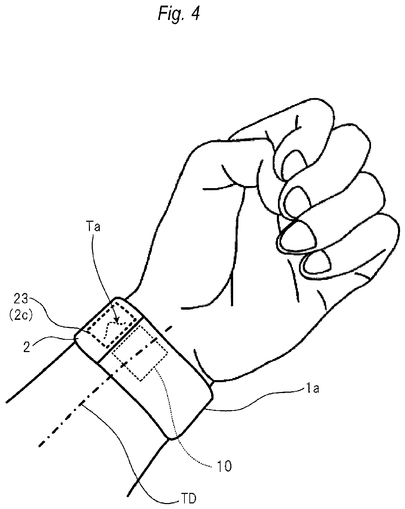

Fig. 4 is a view illustrating a method of attaching the housing 20 to the wrist. In Fig. 4, illustration of components for fixing the housing 20, such as the band is omitted.

The subject causes the detection surface of the pulse wave detection sensor of the pulse wave detector 10 to be contacted with a predetermined position of the wrist which is specified by the manufacturer. As a result of this work, the styloid process Ta is contacted with the bottom surface of the recess 23 of the housing 20.

The recess 23 is configured by the flexible portion 2. Even when the styloid process Ta butts against the bottom surface of the recess 23, therefore, the bottom surface deforms in accordance with the shape of the styloid process Ta, whereby the housing 20 can be prevented from being separated from the wrist H.

Moreover, the bottom surface of the recess 23 is formed by the soft material. Therefore, the subject does not feel a sense of discomfort, and the feeling of attachment is not lowered. In the state of Fig. 4, the subject fixes the housing 20 to the wrist by using the band which is not shown, thereby completing the attachment of the biological information measuring device.

According to the biological information measuring device of the embodiment, as described above, the region 2c with which the styloid process Ta is contacted in the state where the housing 20 is attached to the wrist is configured by the flexible portion 2 that is lower in rigidity than the rigid portion 1a which accommodates the pulse wave detector 10.

Therefore, it is possible to prevent a situation where the housing 20 is caused to enter an unstable state by an influence of the styloid process Ta, from occurring. Consequently, the attachability of the biological information measuring device can be improved. Since the housing 20 is not separated from the wrist, moreover, it is possible to prevent the position of the pulse wave detector 10 from being displaced after attachment, and therefore highly accurate detection of a pressure pulse wave can be performed.

In the biological information measuring device of the embodiment, moreover, the pulse wave detector 10 is accommodated in the rigid portion 1a having a high rigidity. Therefore, it is possible to prevent the position of the pulse wave detector 10 from, after the housing 20 is attached to the wrist, being displaced by an external impact, a motion of the hand, or the like.

In the biological information measuring device of the embodiment, moreover, the recess 23 is formed in the region 2c which is to be contacted with the styloid process Ta. Therefore, the bulge of the styloid process Ta can be absorbed by the recess 23, and the housing 20 can be prevented from separating from the wrist.

The recess 23 may be omitted. Even when the recess 23 is omitted, the region 2c which is to be contacted with the styloid process Ta is configured by the flexible portion 2. Therefore, it is possible to achieve the effects that the attachability is improved, and that the position of the pulse wave detector 10 is prevented from being displaced.

In the state where the housing 20 is wound around the wrist, the position of the region 2c of the housing 20 with which the styloid process Ta is to be contacted is in the vicinity of the interface where the hand palm side changes to the hand back side.

In the biological information measuring device of the embodiment, the portion including the region 2c is configured by the flexible portion 2, and hence the housing 20 is easily bent from the hand palm side to the hand back side. Therefore, the attachment of the housing 20 to the wrist can be easily performed.

Moreover, the degree of close contact to the wrist can be easily adjusted depending on the flexibility of the flexible portion 2, and, irrespective of the shape of the wrist, the attachability of the device can be improved.

In the biological information measuring device of the embodiment, moreover, the thickness of the tip end side of the flexible portion 2 is reduced, and the rigid portion 1b is disposed in the reduced thickness portion.

Therefore, the fixation to the wrist can be stabilized by the rigid portion 1b, and the flexibility of the housing 20 can be increased by the flexible portion 2 which is on the back of the rigid portion 1b, so that the degree of freedom of winding around the wrist can be enhanced. Consequently, both the stable pulse wave detection in the state where the device is attached, and the improvement of the attachability of the device can be attained.

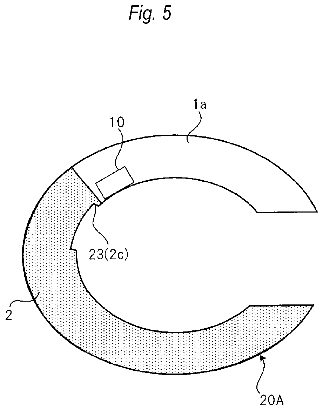

Fig. 5 is a cross-sectional diagram of a housing 20A which is a modification of the housing 20. Fig. 5 is a view corresponding to Fig. 3.

The housing 20A is configured in the same manner as the housing 20 except that the whole rigid portion 1b is changed to the flexible portion 2.

According to the housing 20A, the rigid portion 1a enables the pulse wave detector 10 to be stably fixed to the wrist. Moreover, an unstable state which is caused by the styloid process Ta of the radius T during attachment of the housing 20A to the wrist can be avoided by the flexible portion 2. Since the whole hand back side is configured by the flexible portion 2, moreover, the degree of freedom of attachment of the housing 20 to the wrist can be further enhanced.

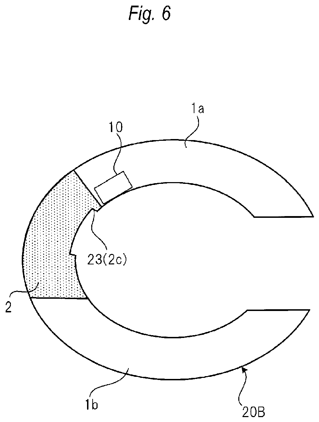

Fig. 6 is a cross-sectional diagram of a housing 20B which is a modification of the housing 20. Fig. 6 is a view corresponding to Fig. 3.

The housing 20B is configured in the same manner as the housing 20 except that the thin portion of the flexible portion 2 is changed to the rigid portion 1b.

According to the housing 20B, the rigid portion 1a and the rigid portion 1b enable the pulse wave detector 10 to be stably fixed to the wrist. Moreover, an unstable state which is caused by the styloid process Ta of the radius T during attachment of the housing 20B to the wrist can be avoided by the flexible portion 2.

Moreover, the degree of freedom of attachment of the housing 20B to the wrist can be enhanced by the flexible portion 2. As compared with the housing 20A, moreover, the rigidity of the whole housing can be increased, and the durability of the device can be enhanced.

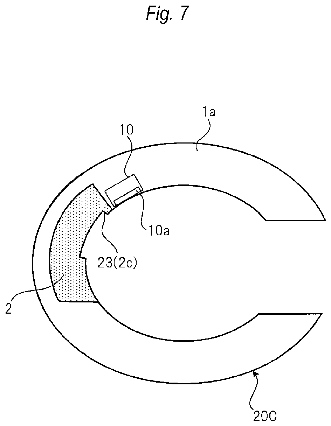

Fig. 7 is a cross-sectional diagram of a housing 20C which is a modification of the housing 20. Fig. 7 is a view corresponding to Fig. 3.

In the housing 20C, an approximately whole of the housing 20 is configured by the rigid portion 1a, and, in the rigid portion 1a, only a portion including the region 2c which is to be contacted with the styloid process Ta is formed as the flexible portion 2. In other words, the housing 20C has a configuration where, in the housing 20B, the outer circumferential surface side of the flexible portion 2 is formed by the rigid portion 1a which is higher in rigidity than the flexible portion 2.

According to the housing 20C, it is possible to achieve the same effects as those of the housing 20B. Moreover, the rigidity of the whole housing can be more increased, and the durability of the device can be further enhanced.

The presently disclosed embodiment should be considered in all respects to be illustrative and not restrictive. The scope of the invention is indicated by the appended claims.

As described above, the following matters are disclosed in the specification.

The disclosed pulse wave detecting device is a pulse wave detecting device which is to be used while being attached to a wrist, wherein the pulse wave detecting device includes a housing which accommodates a pulse wave detection sensor for detecting a pulse wave from a radial artery, the housing includes: a first housing portion; and a second housing portion which is lower in rigidity than the first housing portion, the pulse wave detection sensor is accommodated in the first housing portion, and a region of the housing is configured by at least the second housing portion, the region being contacted with a styloid process of a radius of the wrist in a state where the housing is attached to the wrist.

In the disclosed pulse wave detecting device, the first housing portion includes: a first sub-housing portion which accommodates the pulse wave detection sensor; and a second sub-housing portion, and the housing is configured to be woundable in a circumferential direction of the wrist, and has a structure in which the first sub-housing portion, the second housing portion, and the second sub-housing portion are coupled to one another while being juxtaposed in the circumferential direction.

In the disclosed pulse wave detecting device, the housing is configured to be woundable in a circumferential direction of the wrist, and has a structure in which the first housing portion that accommodates the pulse wave detection sensor, and the second housing portion are coupled to each other while being juxtaposed in the circumferential direction.

In the disclosed pulse wave detecting device, the first housing portion includes: a first sub-housing portion which accommodates the pulse wave detection sensor; and a second sub-housing portion, the first sub-housing portion and the second housing portion are coupled to each other while being juxtaposed in the circumferential direction, and, in a thickness direction of the housing, the second sub-housing portion overlaps with a part of a portion of the second housing portion excluding the region.

In the disclosed pulse wave detecting device, a recess is formed in the region of the housing.

In the disclosed pulse wave detecting device, the second housing portion is configured by an elastomer, rubber, or a sponge.

### Industrial Applicability

According to the invention, it is possible to provide a pulse wave detecting device in which the attachability can be improved while preventing the position of a pulse wave detection sensor from being displaced.

Although the invention has been described with reference to the specific embodiment, the invention is not limited to the embodiment, and various changes can be made. The scope of the invention is defined by the claims.

The application is based on Japanese Patent Application (No. 2015-175966) filed September 7, 2015.

### Reference Signs List

- 1, 1a, 1b: rigid portion
- 2: flexible portion
- 10: pulse wave detector
- 20: housing
- T: radius
- TD: radial artery
- Ta: styloid process
- S: ulna
- H: wrist

## Claims

1. A pulse wave detecting device which includes:
a housing (20) which accommodates a pulse wave detection sensor (10) for detecting a pulse wave from a radial artery; and
a band which is connected to the housing (20) in order to fix the housing to a wrist of a subject, wherein,
the pulse wave detecting device is to be used while being attached to the wrist of the subject so that a part (2c) of the housing (20) contacts with a styloid process (Ta) of a radius (T) of the wrist (H),
**characterized in that**
the housing (20) is configured to be woundable in a circumferential direction of the wrist (H), including a first housing portion (1, 1a, 1b) and a second housing (2) portion which is lower in rigidity than the first housing portion (1, 1a, 1b),
the first housing portion (1, 1a, 1b) and the second housing portion (2) being coupled to each other while being juxtaposed in the circumferential direction, the pulse wave detection sensor is accommodated in the first housing portion (1, 1a, 1b), and
the part (2c) of the housing is configured by at least the second housing (2) portion.

2. The pulse wave detecting device according to claim 1, wherein
the first housing portion (1, 1a, 1b) includes: a first sub-housing (la) portion which accommodates the pulse wave detection sensor; and a second sub-housing portion (1b), and
the housing (20) has a structure in which the first sub-housing portion (la), the second housing portion (2), and the second sub-housing portion (1b) are coupled to one another while being juxtaposed in the circumferential direction.

3. The pulse wave detecting device according to claim 1, wherein
the first housing portion includes (1, 1a, 1b): a first sub-housing portion (la) which accommodates the pulse wave detection sensor; and a second sub-housing portion (1b),
the first sub-housing portion and the second housing portion are coupled to each other while being juxtaposed in the circumferential direction, and,
in a thickness direction of the housing, the second sub-housing portion overlaps with a part of a portion of the second housing portion excluding aregion (B) where the second housing portion contacts with the styloid process of the radius of the wrist.

4. The pulse wave detecting device according to any one of claims 1 to 3, wherein
a recess (23) is formed in the region of the housing where the second housing portion contacts with the styloid process of the radius of the wrist.

5. The pulse wave detecting device according to any one of claims 1 to 4, wherein
the second housing portion (2c) is configured by an elastomer, rubber, or a sponge.

## Patentansprüche

1. Pulswellenerfassungsvorrichtung, aufweisend:
ein Gehäuse (20), das einen Pulswellenerfassungssensor (10) zum Erfassen einer Pulswelle von einer Speichenschlagader aufnimmt; und
ein Band, das mit dem Gehäuse (20) verbunden ist, um das Gehäuse an einem Handgelenk eines Patienten zu befestigen, wobei
die Pulswellenerfassungsvorrichtung zu verwenden ist, während sie an dem Handgelenk des Patienten derart angebracht ist, dass ein Teil (2c) des Gehäuses (20) einen Griffelfortsatz (Ta) einer Speiche (T) des Handgelenks (H) berührt, **dadurch gekennzeichnet, dass**
das Gehäuse (20) eingerichtet ist, um in einer Umfangsrichtung des Handgelenks (H) wickelbar zu sein, aufweisend einen ersten Gehäuseabschnitt (1, 1a, 1b) und einen zweiten Gehäuseabschnitt (2), der eine geringere Steifigkeit aufweist als der erste Gehäuseabschnitt (1, 1a, 1b),
der erste Gehäuseabschnitt (1, 1a, 1b) und der zweite Gehäuseabschnitt (2) miteinander gekoppelt sind, während sie in der Umfangsrichtung nebeneinander liegen,
der Pulswellenerfassungssensor in dem ersten Gehäuseabschnitt (1, 1a, 1b) aufgenommen ist und
der Teil (2c) des Gehäuses durch mindestens den zweiten Gehäuseabschnitt (2) eingerichtet ist.

2. Pulswellenerfassungsvorrichtung nach Anspruch 1, wobei der erste Gehäuseabschnitt (1, 1a, 1b) Folgendes aufweist:
einen ersten Untergehäuseabschnitt (la), der den Pulswellenerfassungssensor aufnimmt; und einen zweiten Untergehäuseabschnitt (1b) und
das Gehäuse (20) eine Struktur aufweist, in welcher der erste Untergehäuseabschnitt (la), der zweite Gehäuseabschnitt (2) und der zweite Untergehäuseabschnitt (1b) miteinander gekoppelt sind, während sie in der Umfangsrichtung nebeneinander liegen.

3. Pulswellenerfassungsvorrichtung nach Anspruch 1, wobei der erste Gehäuseabschnitt (1, 1a, 1b) Folgendes aufweist:
einen ersten Untergehäuseabschnitt (la), der den Pulswellenerfassungssensor aufnimmt; und einen zweiten Untergehäuseabschnitt (1b),
der erste Untergehäuseabschnitt und der zweite Gehäuseabschnitt miteinander gekoppelt sind, während sie in der Umfangsrichtung nebeneinander liegen, und
in einer Dickenrichtung des Gehäuses der zweite Untergehäuseabschnitt mit einem Teil eines Abschnitts des zweiten Gehäuseabschnitts überlappt, ausgenommen einem Bereich (B), in dem der zweite Gehäuseabschnitt den Griffelfortsatz der Speiche des Handgelenks berührt.

4. Pulswellenerfassungsvorrichtung nach einem der Ansprüche 1 bis 3, wobei
eine Vertiefung (23) in dem Bereich des Gehäuses gebildet ist, in dem der zweite Gehäuseabschnitt den Griffelfortsatz der Speiche des Handgelenks berührt.

5. Pulswellenerfassungsvorrichtung nach einem der Ansprüche 1 bis 4, wobei
der zweite Gehäuseabschnitt (2c) durch ein Elastomer, ein Gummi oder einen Schwamm eingerichtet ist.

## Revendications

1. Dispositif de détection d'onde de pouls comprenant :
un boîtier (20) qui héberge un capteur de détection d'onde de pouls (10) pour détecter une onde de pouls à partir d'une artère radiale; et
une bande qui est reliée au boîtier (20) afin de fixer le boîtier à un poignet d'un sujet, dans lequel,
le dispositif de détection d'onde de pouls doit être utilisé en étant fixé au poignet du sujet de sorte qu'une partie (2c) du boîtier (20) est en contact avec un processus styloïde (Ta) d'un rayon (T) du poignet (H),
**caractérisé en ce que**
le boîtier (20) est configuré pour pouvoir être enroulé dans une direction circonférentielle du poignet (H), comprenant une première partie de boîtier (1, 1a, 1b) et une seconde partie de boîtier (2) qui présente une rigidité inférieure à celle de la première partie de boîtier (1, 1a, 1b), la première partie de boîtier (1, 1a, 1b) et la seconde partie de boîtier (2) étant couplées l'une avec l'autre tout en étant juxtaposées dans la direction circonférentielle, le capteur de détection d'onde de pouls est logé dans le la première partie de boîtier (1, 1a, 1b), et la partie (2c) du boîtier est configurée par au moins la seconde partie de boîtier (2).

2. Dispositif de détection d'onde de pouls selon la revendication 1, dans lequel
la première partie de boîtier (1, 1a, 1b) comprend : une première sous-partie de boîtier (1a) qui héberge le capteur de détection d'onde de pouls; et une seconde sous-partie de boîtier (1b), et
le boîtier (20) a une structure dans laquelle la première sous-partie de boîtier (la), la seconde partie de boîtier (2), et la seconde sous-partie de boîtier (1b) sont couplées les unes aux autres tout en étant juxtaposées dans la direction circonférentielle.

3. Dispositif de détection d'onde de pouls selon la revendication 1, dans lequel
la première partie de boîtier comprend (1, 1a, 1b) : une première sous-partie de boîtier (1a) qui héberge le capteur de détection d'onde de pouls; et une seconde sous-partie de boîtier (1b),
la première sous-partie de boîtier et la seconde partie de boîtier sont couplées l'une à l'autre tout en étant juxtaposées dans la direction circonférentielle, et, dans une direction d'épaisseur du boîtier, la seconde sous-partie de boîtier chevauche une partie d'une portion de la seconde partie de boîtier à l'exclusion d'une région (B) où la seconde partie de boîtier est en contact avec le processus styloïde du rayon du poignet.

4. Dispositif de détection d'onde de pouls selon l'une quelconque des revendications 1 à 3, dans lequel
un évidement (23) est formé dans la région du boîtier où la seconde partie de boîtier est en contact avec le processus styloïde du rayon du poignet.

5. Dispositif de détection d'onde de pouls selon l'une quelconque des revendications 1 à 4, dans lequel la seconde partie de boîtier (2c) est constituée d'un élastomère, d'un caoutchouc ou d'une éponge.
